# EUROPEAN PATENT APPLICATION

(11) **EP 0 934 933 A1**
(43) Date of publication of application: **11.08.1999**
(21) Application number: 98102032.4
(22) Date of filing: 06.02.1998
(51) Int. Cl.: C07D 237/32, A61K 31/50

(54) **Phthalazinones**

(71) Applicant: Byk Gulden Lomberg Chemische Fabrik GmbH, 78467 Konstanz (DE)
(72) Inventor: Hatzelmann, Armin, 78467 Konstanz (DE); Boss, Hildegard, 78464 Konstanz (DE); Häfner, Dietrich, 78464 Konstanz (DE); Beume, Rolf, 78465 Konstanz (DE); Kley, Hans-Peter, 78476 Allensbach (DE); Sterk, Geert Jan, 3583 JJ Utrecht (NL); Timmerman, Hendrik, 2253 VM Voorschoten (NL)

(57) **Abstract**

Phthalazinones of the formula I 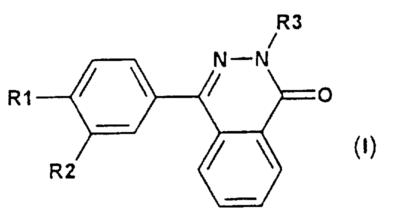 in which R1 is 1-4C alkoxy or 1-4C alkoxy substituted by fluorine, R2 is halogen or (cyclo)alkoxy, and R3 is -CₙH₂ₙ -C(O)R4.

These compounds are suitable as bronchial therapeutics and for the treatment of dermatoses.

## Description

### Field of the application of the invention

The invention relates to novel Phthalazinones, which are used in the pharmaceutical industry for the production of medicaments.

### Known technical background

International Patent Application WO91/12251 describes phthalazinones having bronchodilating and thromboxane A2 synthase inhibiting properties. International Patent Application WO93/07146 describes benzo- and pyrido-pyridazinone and -pyridazinthione compounds with PDE4 inhibiting activity.

### Description of the invention

It has now been found that the phthalazinones, which are described in greater detail below, have surprising and particularly advantages properties.

The invention thus relates to compounds of the formula I 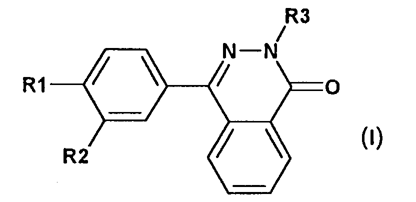 in which
- R1: is 1-4C-alkoxy or 1-4C-alkoxy which is completely or predominantly substituted by fluorine,
- R2: is halogen, 1-8C-alkoxy, 3-7C-cycloalkoxy, 3-7C-cycloalkylmethoxy, or 1-4C-alkoxy which is completely or predominantly substituted by fluorine,
- R3: is-CₙH₂ₙ-C(O)R4, in which
- R4: is phenyl, naphthyl, pyridyl, or phenyl substituted by R41, R42 and R43, where
- R41: is hydroxyl, halogen, cyano, 1-4C-alkyl, 1-4C-alkoxy, carboxyl, 1-4C-alkoxycarbonyl, aminocarbonyl, mono- or di-1-4C-alkylaminocarbonyl, amino, mono- or di-1-4C-alkylamino, 1-4C-alkylcarbonylamino, or 1-4C-alkoxy which is completely or predominantly substituted by fluorine, and
- R42: is hydrogen, halogen, 1-4C-alkyl or 1-4C-alkoxy,
- R43: is hydrogen, halogen, 1-4C-alkyl or 1-4C-alkoxy,
- n: is an integer from 1 to 4,
and the salts of these compounds.

1-4C-Alkoxy is a radical, which, in addition to the oxygen atom contains a straight-chain or branched alkyl radical having 1 to 4 carbon atoms. Alkoxy radicals having 1 to 4 carbon atoms which may be mentioned in this context are, for example, the butoxy, iso-butoxy, sec-butoxy, tert-butoxy, propoxy and in particular the isopropoxy, ethoxy and methoxy radicals.

1-4C-Alkoxy which is completely or predominantly substituted by fluorine is, for example, the 2,2,3,3,3-pentafluoropropoxy, the perfluoroethoxy, the 1,2,2-trifluoroethoxy and in particular the 1,1,2,2-tetrafluoroethoxy, the 2,2,2-trifluoroethoxy, the trifluoromethoxy and the difluoromethoxy radical, of which the difluoromethoxy radical is preferred.

1-8C-Alkoxy is a radical which, in addition to the oxygen atom, contains a straight-chain or branched alkyl radical having 1 to 8 carbon atoms. Alkoxy radicals having 1 to 8 carbon atoms which may be mentioned in this context are, for example, the octyloxy, heptyloxy, isoheptyloxy (5-methylhexyloxy), hexyloxy, isohexyloxy (4-methylpentyloxy), neohexyloxy (3,3-dimethylbutoxy), pentyloxy, isopentyloxy (3-methylbutoxy), neopentyloxy (2,2-dimethylpropoxy), butoxy, isobutoxy, sec-butoxy, tert-butoxy, propoxy and in particular the isopropoxy, ethoxy and methoxy radicals.

3-7C-Cycloalkoxy stands for cyclopropyloxy, cyclobutyloxy, cyclopentyloxy, cyclohexyloxy or cycloheptyloxy, of which cyclopropyloxy, cyclobutyloxy and cyclopentyloxy are preferred.

3-7C-Cycloalkylmethoxy stands for cyclopropylmethoxy, cyclobutylmethoxy, cyclopentylmethoxy, cyclohexylmethoxy or cycloheptylmethoxy, of which cyclopropylmethoxy, cyclobutylmethoxy and cyclopentylmethoxy are preferred.

1-4C-Alkoxycarbonyl is a carbonyl group to which one of the abovementioned 1-4C-alkoxy radicals is bonded. Examples are the methoxycarbonyl (CH₃O-C(O)-) and the ethoxycarbonyl (CH₃CH₂O-C(O)-) radical.

Mono- or Di-1-4C-alkylamino radicals contain in addition to the nitrogen atom, one or two of the abovementioned 1-4C-alkyl radicals. Preferred are the di-1-4C-alkylamino radicals, especially the dimethylamino, the diethylamino and the diisopropylamino radical.

Mono- or Di-1-4C-alkylaminocarbonyl radicals contain in addition to the carbonyl group one of the abovementioned mono- or di-1-4C-alkylamino radicals. Examples which may be mentioned are the N-methyl- the N,N-dimethyl-, the N-ethyl-, the N-propyl-, the N,N-diethyl- and the N-isopropylaminocarbonyl radical.

An 1-4C-Alkylcarbonylamino radical is, for example, the propionylamino (C₃H₇C(O)NH-) and the acetylamino radical (CH₃C(O)NH-).

The group -CₙH₂ₙ- can be a straight chain or a branched group. Examples which may be mentioned are the butylene, isobutylene, propylene, isopropylene, ethylene, 1-methylmethylene and the methylene group.

Suitable salts for compounds of the formula I - depending on substitution - are all acid addition salts or all salts with bases. Particular mention may be made of the pharmacologically tolerable salts with the inorganic and organic acids and bases customarily used in pharmacy. Those suitable are, on the one hand, water-soluble and water-insoluble acid addition salts with acids such as, for example, hydrochloric acid, hydrobromic acid, phosphoric acid, nitric acid, sulphuric acid, acetic acid, citric acid, D-gluconic acid, benzoic acid, 2-(4-hydroxybenzoyl)benzoic acid, butyric acid, sulphosalicylic acid, maleic acid, lauric acid, malic acid, fumaric acid, succinic acid, oxalic acid, tartaric acid, embonic acid, stearic acid, toluenesulphonic acid, methanesulphonic acid or 3-hydroxy-2-naphthoic acid, the acids being employed in salt preparation - depending on whether a mono- or polybasic acid is concerned and depending on which salt is desired - in an equimolar quantitative ratio or one differing therefrom.

On the other hand, salts with bases are - depending on substitution - also suitable. As examples of salts with bases are mentioned the lithium, sodium, potassium, calcium, aluminum, magnesium, titanium, ammonium, meglumine or guanidinium salts, here too, the bases being employed in salt preparation in an equimolar quantitative ratio or one differing therefrom.

Pharmacologically intolerable salts, which can be obtained, for example, as process products during the preparation of the compounds according to the invention on an industrial scale, are converted into pharmacologically tolerable salts by processes known to the person skilled in the art.

According to expert's knowledge the compounds of the invention as well as their salts may contain, e.g. when isolated in crystalline form, varying amounts of solvents. Included within the scope of the invention are therefore all solvates and in particular all hydrates of the compounds of formula I as well as all solvates and in particular all hydrates of the salts of the compounds of formula I.

Compounds of the formula I to be emphasized are those, in which
- R1: is 1-2C-alkoxy or 1-2C-alkoxy which is completely or predominantly substituted by fluorine,
- R2: is 1-4C-alkoxy, 3-7C-cycloalkoxy, 3-7C-cycloalkylmethoxy, or 1-4C-alkoxy which is completely or predominantly substituted by fluorine,
- R3: is -CₙH₂ₙ-C(O)R4, in which
- R4: is phenyl, naphthyl, or phenyl substituted by R41, R42 and R43, where
- R41: is hydroxyl, halogen, 1-4C-alkyl, 1-4C-alkoxy, carboxyl, aminocarbonyl, amino or mono-or di-1-4C-alkylamino,
- R42: is hydrogen, halogen, 1-4C-alkyl or 1-4C-alkoxy,
- R43: is hydrogen or halogen,
- n: is an integer from 1 to 2,
and the salts of these compounds.

Compounds of the formula I, which are particularly to be emphasized are those, in which
- R1: is 1-2C-alkoxy,
- R2: is 1-2C-alkoxy, 3-5C-cycloalkoxy, 3-5C-cycloalkylmethoxy, or 1-2C-alkoxy which is completely or predominantly substituted by fluorine,
- R3: is - CₙH₂ₙ-C(O)R4, in which
- R4: is phenyl, naphthyl, or phenyl substituted by R41, R42 and R43, where
- R41: is halogen, 1-4C-alkyl, 1-4C-alkoxy or amino,
- R42: is hydrogen, halogen or 1-4C-alkoxy,
- R43: is hydrogen or halogen,
- n: is an integer from 1 to 2,
and the salts of these compounds.

Preferred compounds of formula I are those, in which
- R1: is methoxy or ethoxy,
- R2: is methoxy, ethoxy, difluoromethoxy or cyclopentyloxy,
- R3: is -CₙH₂ₙ-C(O)R4, in which
- R4: is phenyl, naphthyl, or phenyl substituted by R41, R42 and R43, where
- R41: is amino, chlorine, methyl or methoxy,
- R42: is hydrogen, chlorine or methoxy,
- R43: is hydrogen or chlorine,
- n: is 1,
and the salts of these compounds.

The invention further relates to a process for the preparation of compounds of formula I and their salts.

The process comprises reacting keto acids of formula II 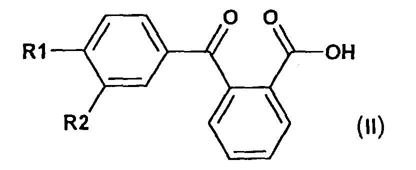 or one of their reactive derivatives in which R1 and R2 have the abovementioned meanings in a first step with hydrazine hydrate to compounds of formula I 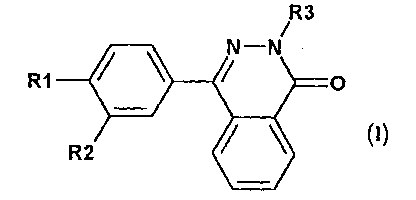 in which R1 and R2 have the abovementioned meanings and R3 stands for hydrogen (H).

These compounds can be further reacted with alkylating agents of formula R3-X, in which R3 has the abovementioned meanings [exception: R3 does not represent hydrogen (H)] and X represents a leaving group to give further compounds of formula I, in which R1, R2 and R3 have the abovementioned meanings [exception: R3 does not represent hydrogen (H)].

The conversion of the keto acids of formula II or one of their reactive derivatives with hydrazine hydrate is advantageously carried out with one to five equivalents of hydrazine hydrate with or without an additional solvent. More suitable is, however, to use an additional appropriate solvent. As inert solvents are preferably used alcohols such as methanol, ethanol, isopropanol, n-butanol, isoamylalcohol, glycols and their ethers such as ethylene glycol, diethylene glycol, ethylene glycol, monomethyl or monoethyl ether, carboxylic acids, such as formic acid, acetic or propionic acid, suitable mixtures of the abovementioned solvents, as well as mixtures with water, for example aqueous ethanol, further ethers, especially water soluble ethers such as tetrahydrofuran, dioxane or ethylene glycol dimethylether; further toluene or benzene, especially when the method of azeotropic destillation is used to remove the reaction water.

The reaction temperatures are suitably between -20 and 120°C, preferably between 20 and 100°C; the reaction times are preferably between 1 and 48 hours.

Suitable reactive derivatives of the keto acids of formula II which may be mentioned in this context are, for example, esters, especially methyl and ethyl esters, nitrils and acid halides, such as acid chlorides or acid bromides. They can be prepared, for example, starting from the corresponding keto acids of formula II, by methods which are known by the person skilled in the art.

The conversion of compounds of formula I in which R1 and R2 have the abovementioned meanings and R3 represents hydrogen (H) with alkylating agents of the formula R3-X, in which R3 has the abovementioned meanings [with the exception of hydrogen(H)] and X represents a suitable leaving group, is carried out in a manner, which is known by a person skilled in the art.

In a first step, the hydrogen atom (H) of the NH-group of the compounds of formula I, in which R3 represents a hydrogen atom (H) is removed by a base such as, for example, potassium carbonate, sodium hydroxide, sodium hydride, sodium methanolate, sodium ethanolate or triethylamine in a suitable inert solvent such as dimethylformamide, dimethylsulfoxide, toluene, tetrahydrofuran or diethylether.

The alkylation is then carried out by adding an appropriate alkylating agent of the formula R3-X.

The bases are preferably used in more than an equimolar ratio; the reaction temperature is preferably between 20 and 100°C.

Examples of suitable leaving groups X which may be mentioned are halogen atoms, especially chlorine, or hydroxyl groups activated by esterification (for example with p-toluenesulfonic acid).

Suitable alkylating agents of the formula R3-X are for example ω-bromoacetophenone, ω-bromo-4-methylacetophenone, ω-bromo-4-chloroacetophenone, ω-bromo-2-methoxyacetophenone, ω-bromo-3-methoxyacetophenone, ω-bromo-4-methoxyacetophenone, ω-bromo-4-amino-3,5-dichloroacetophenone, 2-bromopropiophenone, ω-bromo-2-acetonaphthone and ω-bromo-1-acetonaphthone.

Keto acids of the formula II, in which R1 and R2 have the abovementioned meanings can, for example, be prepared from compounds of the formula III, 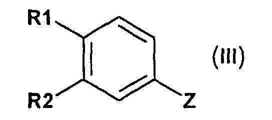 in which R1 and R2 have the abovementioned meanings and Z represents hydrogen (H) by Friedel-Crafts acylation with phthalic acid anhydride.

The Friedel-Crafts acylation is carried out in a manner which in known by the skilled person (for example as described in M. Yamaguchi et a., J. Med. Chem. 36, 4052-4060,1993 or in A. Oliverio and E. Lugli, Gazz. Chim Ital. 78, 16-20, 1948) in presence of a suitable catalyst, such as for example, AlCl₃, ZnCl₂, FeCl₃ or iodine, in an appropriate inert solvent, such as methylene chloride or nitrobenzene or another inert solvent such as diethylether, preferably at raised temperature, especially at the boiling point of the solvent being used.

Alternatively, the compounds of formula II, in which R1 and R2 have the abovementioned meanings, can be prepared from compounds of the formula III, in which R1 and R2 have the abovementioned meanings and Z represents a halogen atom through reaction with phthalic acid anhydride.

The reaction, which is mentioned in the previous paragraph is carried out in a manner which is known by a person skilled in the art, for example
a) by activating compounds of formula III, in which R1, R2 and Z have the abovementioned meanings, by a lithium/halogen exchange reaction at low temperatures (preferably at - 60 to -100°C) in an appropriate inert solvent such as tetrahydrofuran or diethylether, preferably under an atmosphere of inert gas, followed by reaction of the lithiated compounds with phthalic acid anhydride, or
b) by converting compounds of formula III in which R1, R2 and Z have the abovementioned meanings, in a suitable inert solvent such as, for example, tetrahydrofuran or diethylether into the corresponding Grignard compounds of formula III in which Z represents MgCl, MgBr or Mgl followed by reaction of the Grignard compounds with phthalic acid anhydride.

Compounds of formula III, in which R1 and R2 have the abovementioned meanings and Z represents a hydrogen (H) or halogen atom, are known or can be prepared by methods known by a person skilled in the art.

Additionally, it is possible to convert one functional group of a compound of formula I to another functional group by customary methods and reactions.

Thus, if desired, compounds of formula I with suitable functional groups can be converted into further compounds of formula I.

For instance, compounds of formula I, in which R3 comprises an ester can be converted by acidic or alkaline saponification to the corresponding carboxylic acid or by reaction with a suitable amine to the corresponding amide.

Furthermore, compounds of formula I, in which R2 comprises an acid able ether bond, such as for example, the cyclopentyl-oxygen bond, can be converted by an acidic ether cleavage reaction into compounds of formula I, in which R2 represents a hydroxyl group. The renewed alkylation of this hydroxyl group with appropriate alkylation reagents leads to further compounds of formula I.

As appropriate alkylation reagents may be mentioned in this context, for example, chlorodifluoromethane, ethyliodide or cyclopropylmethylchloride.

Suitably, the conversions are carried out analogous to methods which are familiar per se to the person skilled in the art, for example, in the manner which is described in the following examples.

The substances according to the invention are isolated and purified in a manner known per se, e.g. by distilling off the solvent in vacuo and recrystallizing the residue obtained from a suitable solvent or subjecting it to one of the customary purification methods, such as column chromatography on a suitable support material.

Salts are obtained by dissolving the free compound in a suitable solvent, e.g. in a chlorinated hydrocarbon, such as methylene chloride or chloroform, or a low molecular weight aliphatic alcohol (ethanol, isopropanol) which contains the desired acid or base, or to which the desired acid or base is then added. The salts are obtained by filtering, reprecipitating, precipitating with a non-solvent for the addition salt or by evaporating the solvent. Salts obtained can be converted by basification or by acidifying into the free compounds which, in turn, can be converted into salts. In this manner, pharmacologically non-tolerable salts can be converted into pharmacologically tolerable salts.

The following examples illustrate the invention in greater detail, without restricting it. As well, further compounds of formula I, of which the preparation is explicitly not described, can be prepared in an analogous way or in a way which is known by a person skilled in the art using customary preparation methods.

The compounds, which are mentioned in the examples as well as their salts are preferred compounds of the invention.

### Examples

### Final Products

### 1. 2-(2-Phenyl-2-oxoethyl)-4-(3,4-dimethoxyphenyl)-2H-phthalazin-1-one

A mixture of 1.4 g of compound B (see starting compounds), 1.0 g of potassium carbonate and 1.0 g of ω-chloroacetophenone was heated at 110°C. After 8 h, the mixture was evaporated and the residue partitioned between ethyl acetate and water. The organic layer was dried over magnesium sulfate and evaporated. Purification by chromatography [ethyl acetate: petroleum ether (60-80°C), 1:2]. Crystallized from ethyl acetate/petroleum ether (60-80°C). M. p. 196-199°C

### 2. 2-(2-Phenyl-2-oxoethyl)-4-(3,4-diethoxyphenyl)-2H-phthalazin-1-one

Prepared from compound D and ω-bromoacetophenone as described for compound 1. Crystallized from methanol. M. p. 174-175°C

### 3. 4-(3-Ethoxy-4-methoxyphenyl)-2-(2-phenyl-2-oxoethyl)-2H-phthalazin-1-one

A solution of 1 g of compound G, 1 g of ethyl iodide and 1 g of potassium carbonate in 10 ml of dimethyl formamide was stirred at 80°C for 3 h. After cooling to room temperature, ethyl acetate was added and the resulting mixture washed with water. After drying over magnesium sulfate and evaporation of the ethyl acetate the compound was crystallized from ethyl acetate/petroleum ether (60-80°C). M. p. 136-138°C

### 4. 4-(3-Cyclopentyloxy-4-methoxyphenyl)-2-(2-phenyl-2-oxoethyl)-2H-phthalazin-1-one

Prepared from compound F (see starting compounds) and ω-bromoacetophenone as described for compound 1. Crystallized from ethyl acetate. M. p. 185-186°C

### 5. 4-(3-Difluoromethoxy-4-methoxyphenyl)-2-(2-phenyl-2-oxoethyl)-2H-phthalazin-1-one

To a solution of 3.4 g of compound G (see starting compounds), 0.2 g of tetraethylammonium bromide and 1.1 g of sodium hydroxide in 1 ml of water in 13 ml of dioxane at 80°C, a flow of chlorodifluoromethane was introduced for 30 min. The solution was kept at a pH between 9 and 11 throughout the reaction by addition of small amounts of a 6 N sodium hydroxide solution. After evaporating the reaction mixture the residue was partitioned between water and ethyl acetate. The organic layer was dried over magnesium sulfate and evaporated. The compound was purified by chromatography [ethyl acetate/petroleum ether (60-95°C), 5:2] and crystallized from diethyl ether. M. p. 132-133°C

### 6. 4-(3,4-Dimethoxyphenyl)-2-[2-(4-methylphenyl)-2-oxoethyl]-2H-phthalazin-1-one

Prepared from compound B and ω-bromo-4-methylacetophenone as described for compound 1. Crystallized from methanol. M. p. 175-177°C

### 7. 4-(3,4-Dimethoxyphenyl)-2-[2-(2-methoxyphenyl)-2-oxoethyl]-2H-phthalazin-1-one

Prepared from compound B and ω-bromo-2-methoxyacetophenone as described for compound 1. Crystallized from diethyl ether. M. p. 128-130°C

### 8. 4-[3,4-Dimethoxyphenyl)-2-[2-(3-methoxyphenyl)-2-oxoethyl]-2H-phthalazin-1-one

Prepared from compound B and ω-bromo-3-methoxyacetophenone as described for compound 1. Crystallized from diethyl ether. M. p. 125-126°C

### 9. 4-(3,4-Dimethoxyphenyl)-2-[2-(4-methoxyphenyl)-2-oxoethyl]-2H-phthalazin-1-one

Prepared from compound B and ω-bromo-4-methoxyacetophenone as described for compound 1. Crystallized from diethyl ether. M. p. 179-181 °C

### 10. 4-(3,4-Dimethoxyphenyl)-2-[2-(2,5-dimethoxyphenyl)-2-oxoethyl]-2H-phthalazin-1-one

Prepared from compound B and ω-bromo-2,5-dimethoxyacetophenone as described for compound 1. Crystallized from diethyl ether. M. p. 130-132°C

### 11. 4-(3,4-Dimethoxyphenyl)-2-[2-(4-chlorophenyl)-2-oxoethyl]-2H-phthalazin-1-one

Prepared from compound B and ω-bromo-4-chloroacetophenone as described for compound 1. Crystallized from methanol. M. p. 192-194°C

### 12. 4-(3,4-Dimethoxyphenyl)-2-[2-(4-amino-3,5-dichlorophenyl)-2-oxoethyl]-2H-phthalazin-1-one

Prepared from compound B and 4-amino-ω-bromo-3,5-dichloroacetophenone as described for compound 1. Crystallized from diethyl ether. M. p. 226-228°C

### 13. 4-(3,4-Dimethoxyphenyl)-2-(2-phenyl-2-oxo-1-methylethyl]-2H-phthalazin-1-one

Prepared from compound B and 2-bromopropiophenone as described for compound 1. Crystallized from methanol. M. p. 150-152°C

### 14. 2-[2-(2-Naphthyl)-2-oxoethyl]-4-(3,4-dimethoxyphenyl)-2H-pthalazin-1-one

Prepared from compound B and ω-bromo-2-acetonaphthone as described for compound 1. Crystallized from methanol. M. p. 186-189°C

### 15. 2-[2-(1-Naphthyl)-2-oxoethyl]-4-(3,4-dimethoxyphenyl)-2H-phthalazin-1-one

Prepared from compound B and ω-bromo-1-acetonaphthone as described for compound 1. Purified by chromatography [petroleum ether (60-80°C)/ethyl acetate, 2:1]. Crystallized from ethyl acetate/petroleum ether (60-80°C). M. p. 168-169°C

### Starting compounds

### A. 2-(3,4-Dimethoxybenzoyl)benzoic acid

Prepared from phthalic acid anhydride and 1,2-dimethoxybenzene as described by A. Oliverio and E. Lugli in Gazz. Chim. Ital. 78, 16-20 (1948).

### B. 4-(3,4-Dimethoxyphenyl]-2H-phthalazin-1-one

A solution of 20 g of compound A and 20 ml of hydrazine hydrate in 200 ml of ethanol was refluxed for 3 h. After cooling to room temperature the precipitate was filtered off and dried. M. p. 244-249°C

### C. 2-(3,4-Diethoxybenzoyl)benzoic acid

Prepared from phthalic anhydride and 1,2-diethoxybenzene as described for compound A.

### D. 4-(3,4-Diethoxyphenyl)-2H-phthalazin-1-one

Prepared from compound C and hydrazine hydrate as described for compound B. M. p. 211-212°C

### E. 2-(3-Cyclopentyloxy-4-methoxybenzoyl)benzoic acid

10.5 g (38.7 mmol) of 1-bromo-3-cyclopentyloxy-4-methoxybenzene was dissolved in 100 ml of freshly destilled tetrahydrofuran and cooled to -80°C. 25 ml of butyllithium (1.6 M in hexane) was added dropwise. The reaction mixture was added to a solution of 6.32 g (42.7 mmol) of phthalic anhydride in 100 ml of tetrahydrofuran (-80°C). The reaction mixture was stirred for 2 h. Ammonium chloride was added and the reaction mixture was warmed to 8°C and then water was added. The layers were separated, the water layer was extracted with ethyl acetate. The combined organic layers were dried over magnesium sulfate and evaporated. Purified by chromatography [petroleum ether (60-95°C)/ethyl acetate, 1:1]. crystallized from ethyl acetate/petroleum ether (60-95°C). M. p. 123-125°C

### F. 4-(3-Cyclopentyloxy-4-methoxy)-2H-phthalazin-1-one

Prepared from compound E and hydrazine hydrate as described for compound B. M. p. 207-208°C

### G. 4-(3-Hydroxy-4-methoxyphenyl)-2-(2-phenyl-2-oxoethyl)-2H-phthalazin-1-one

A solution of 5 g of compound 1 and 5 g of p-toluenesulphonic acid in 100 ml of toluene was heated in a Dean-Stark apparatus for 6 h. After evaporating the reaction mixture, the residue was partitioned between aqueous sodium carbonate and ethyl acetate. The ethyl acetate solution was dried over magnesium sulfate and evaporated. The compound was crystallized from diethyl ether. M. p. 160-161°C

### Commercial utility

The compounds according to the invention have useful pharmacological properties which make them industrially utilizable. As selective cyclic nucleotide phosphodiesterase (PDE) inhibitors (specifically of type 4), they are suitable on the one hand as bronchial therapeutics (for the treatment of airway obstructions on account of their dilating action but also on account of their respiratory rate-or respiratory drive-increasing action) and for the removal of erectile dysfunction on account of their vascular dilating action, but on the other hand especially for the treatment of disorders, in particular of an inflammatory nature, e.g. of the airways (asthma prophylaxis), of the skin, of the intestine, of the eyes, of the CNS and of the joints, which are mediated by mediators such as histamine, PAF (platelet-activating factor), arachidonic acid derivatives such as leukotrienes and prostaglandins, cytokines, interleukins, chemokines, alpha-, beta- and gamma-interferon, tumor necrosis factor (TNF) or oxygen free radicals and proteases. In this context, the compounds according to the invention are distinguished by a low toxicity, a good enteral absorption (high bioavailability), a large therapeutic breadth and the absence of significant side effects.

On account of their PDE-inhibiting properties, the compounds according to the invention can be employed in human and veterinary medicine as therapeutics, where they can be used, for example, for the treatment and prophylaxis of the following illnesses: acute and chronic (in particular inflammatory and allergen-induced) airway disorders of varying origin (bronchitis, allergic bronchitis, bronchial asthma); dermatoses (especially of proliferative, inflammatory and allergic type) such as psoriasis (vulgaris), toxic and allergic contact eczema, atopic eczema, seborrhoeic eczema, Lichen simplex, sunburn, pruritus in the anogenital area, alopecia areata, hypertrophic scars, discoid lupus erythematosus, follicular and widespread pyodermias, endogenous and exogenous acne, acne rosacea and other proliferative, inflammatory and allergic skin disorders; disorders which are based on an excessive release of TNF and leukotrienes, for example disorders of the arthritis type (rheumatoid arthritis, rheumatoid spondylitis, osteoarthritis and other arthritic conditions), disorders of the immune system (AIDS, multiple sclerosis), types of shock (septic shock, endotoxin shock, gram-negative sepsis, toxic shock syndrome and ARDS (adult respiratory distress syndrome)) and also generalized inflammations in the gastrointestinal region (Crohn's disease and ulcerative colitis); disorders which are based on allergic and/or chronic, immunological false reactions in the region of the upper airways (pharynx, nose) and the adjacent regions (paranasal sinuses, eyes), such as allergic rhinitis/sinusitis, chronic rhinitis/sinusitis, allergic conjunctivitis and also nasal polyps; but also disorders of the heart which can be treated by PDE inhibitors, such as cardiac insufficiency, or disorders which can be treated on account of the tissue-relaxant action of the PDE inhibitors, such as, for example, erectile dysfunction or colics of the kidneys and of the ureters in connection with kidney stones; and also illnesses of the central nervous system, such as depressions or arteriosclerotic dementia.

The invention further relates to a method for the treatment of mammals, including humans, which are suffering from one of the abovementioned illnesses. The method is characterized in that a therapeutically active and pharmacologically effective and tolerable amount of one or more of the compounds according to the invention is administered to the ill mammal.

The invention further relates to the compounds according to the invention for use in the treatment and/or prophylaxis of the illnesses mentioned.

The invention also relates to the use of the compounds according to the invention for the production of medicaments which are employed for the treatment and/or prophylaxis of the illnesses mentioned.

The invention furthermore relates to medicaments for the treatment and/or prophylaxis of the illnesses mentioned, which contain one or more of the compounds according to the invention.

The medicaments are prepared by processes which are known per se and familiar to the person skilled in the art. As medicaments, the compounds according to the invention (= active compounds) are either employed as such, or preferably in combination with suitable pharmaceutical auxiliaries, e.g. in the form of tablets, coated tablets, capsules, suppositories, patches, emulsions, suspensions, gels or solutions, the active compound content advantageously being between 0.1 and 95%.

The person skilled in the art is familiar with auxiliaries which are suitable for the desired pharmaceutical formulations on account of his expert knowledge. In addition to solvents, gel formers, ointment bases and other active compound excipients, for example antioxidants, dispersants, emulsifiers, preservatives, solubilizers or permeation promoters, can be used.

For the treatment of disorders of the respiratory tract, the compounds according to the invention are preferably also administered by inhalation. To do this, these are either administered directly as a powder (preferably in micronized form) or by atomizing solutions or suspensions which contain them. With respect to the preparations and administration forms, reference is made, for example, to the details in European Patent 163 965.

For the treatment of dermatoses, the compounds according to the invention are in particular administered in the form of those medicaments which are suitable for topical application. For the production of the medicaments, the compounds according to the invention (= active compounds) are preferably mixed with suitable pharmaceutical auxiliaries and further processed to give suitable pharmaceutical formulations. Suitable pharmaceutical formulations are, for example, powders, emulsions, suspensions, sprays, oils, ointments, fatty ointments, creams, pastes, gels or solutions.

The medicaments according to the invention are prepared by processes known per se. The dosage of the active compounds is carried out in the order of magnitude customary for PDE inhibitors. Topical application forms (such as ointments) for the treatment of dermatoses thus contain the active compounds in a concentration of, for example, 0.1-99%. The dose for administration by inhalation is customarily between 0.1 and 3 mg per day. The customary dose in the case of systemic therapy (p.o. or i.v.) is between 0.03 and 3 mg/kg.

### Biological investigations

In the investigation of PDE 4 inhibition on the cellular plane, the activation of inflammatory cells is ascribed particular importance. An example is FMLP (N-formyl-methionyl-leucyl-phenylalanine)-induced superoxide production of neutrophilic granulocytes, which can be measured as luminol-amplified chemiluminescence. (Mc Phail LC, Strum SL, Leone PA and Sozzani S, The neutrophil respiratory burst mechanism. In "Immunology Series" 57: 47-76, 1992; ed. Coffey RG (Marcel Decker, Inc., New York-Basel-Hong Kong)).

Substances which inhibit chemiluminescence and cytokine secretion and the secretion of proinflammatory mediators on inflammatory cells, in particular neutrophilic and eosinophilic granulocytes, T-lymphocytes, monocytes and macrophages are those which inhibit PDE 4. This isoenzyme of the phosphodiesterase families is particularly represented in granulocytes. Its inhibition leads to an increase in the intracellular cyclic AMP concentration and thus to the inhibition of cellular activation. PDE 4 inhibition by the substances according to the invention is thus a central indicator for the suppression of inflammatory processes. (Giembycz MA, Could isoenzyme-selective phosphodiesterase inhibitors render bronchodilatory therapy redundant in the treatment of bronchial asthma?. Biochem Pharmacol 43: 2041-2051, 1992; Torphy TJ et al., Phosphodiesterase inhibitors: new opportunities for treatment of asthma. Thorax 46: 512-523, 1991; Schudt C et al., Zardaverine: a cyclic AMP PDE 3/4 inhibitor. In "New Drugs for Asthma Therapy", 379-402, Birkhäuser Verlag Basel 1991; Schudt C et al., Influence of selective phosphodiesterase inhibitors on human neutrophil functions and levels of cAMP and Ca; Naunyn-Schmiedebergs Arch Pharmacol 344; 682-690, 1991; Nielson CP et al., Effects of selective phosphodiesterase inhibitors on polymorphonuclear leucocyte respiratory burst. J Allergy Clin Immunol 86: 801-808, 1990; Schade et al., The specific type 3 and 4 phosphodiesterase inhibitor zardaverine suppresses formation of tumor necrosis factor by macrophages. European Journal of Pharmacology 230: 9-14, 1993).

### Inhibition of PDE 4 activity

### Methodology

The activity test was carried out according to the method of Bauer and Schwabe, which was adapted to microtitre plates (Naunyn-Schmiederberg's Arch. Pharmacol. 311, 193-198, 1980). In this test, the PDE reaction is carried out in the first step. In a second step, the resultant 5'-nucleotide is cleaved to the uncharged nucleoside by a snake venom 5'-nucleotidase from Crotalus Atrox. In the third step, the nucleoside is separated from the remaining charged substrate on ion exchange columns. The columns are eluted directly into minivials using 2 ml of 30 mM ammonium formate (pH 6.0), to which a further 2 ml of scintillation fluid is added for counting.

The inhibitory values determined for the compounds according to the invention follow from the following table A, in which the numbers of the compounds correspond to the numbers of the examples.

**Table A**

| Inhibition of PDE4 activity [measured as -logIC₅₀ (mol/l)] | |
|---|---|
| **Compound** | **-logIC**_{**50**} |
| 2 | 7.01 |
| 3 | 7.28 |
| 4 | 7.09 |
| 5 | 7.14 |
| 7 | 7.52 |
| 10 | 6.99 |
| 12 | 6.85 |

## Claims

1. Compounds of the formula I 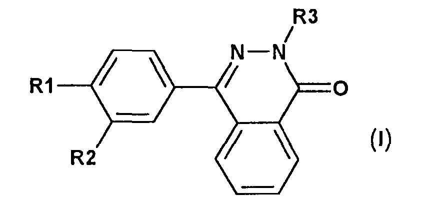 in which
R1 is 1-4C-alkoxy or 1-4C-alkoxy which is completely or predominantly substituted by fluorine,
R2 is halogen, 1-8C-alkoxy, 3-7C-cycloalkoxy, 3-7C-cycloalkylmethoxy, or 1-4C-alkoxy which is completely or predominantly substituted by fluorine,
R3 is -CₙH₂ₙ-C(O)R4, in which
R4 is phenyl, naphthyl, pyridyl, or phenyl substituted by R41, R42 and R43, where
R41 is hydroxyl, halogen, cyano, 1-4C-alkyl, 1-4C-alkoxy, carboxyl, 1-4C-alkoxycarbonyl, aminocarbonyl, mono- or di-1-4C-alkylaminocarbonyl, amino, mono- or di-1-4C-alkylamino, 1-4C-alkylcarbonylamino, or 1-4C-alkoxy which is completely or predominantly substituted by fluorine, and
R42 is hydrogen, halogen, 1-4C-alkyl or 1-4C-alkoxy,
R43 is hydrogen, halogen, 1-4C-alkyl or 1-4C-alkoxy,
n is an integer from 1 to 4,
and the salts of these compounds.

2. Compounds of the formula I according to claim 1, in which
R1 is 1-2C-alkoxy or 1-2C-alkoxy which is completely or predominantly substituted by fluorine,
R2 is 1-4C-alkoxy, 3-7C-cycloalkoxy, 3-7C-cycloalkylmethoxy, or 1-4C-alkoxy which is completely or predominantly substituted by fluorine,
R3 is -CnH₂ₙ-C(O)R4, in which
R4 is phenyl, naphthyl, or phenyl substituted by R41, R42 and R43, where
R41 is hydroxyl, halogen, 1-4C-alkyl, 1-4C-alkoxy, carboxyl, aminocarbonyl, amino or mono-or di-1-4C-alkylamino,
R42 is hydrogen, halogen, 1-4C-alkyl or 1-4C-alkoxy,
R43 is hydrogen or halogen,
n is an integer from 1 to 2,
and the salts of these compounds.

3. Compounds of the formula I according to claim 1, in which
R1 is 1-2C-alkoxy,
R2 is 1-2C-alkoxy, 3-5C-cycloalkoxy, 3-5C-cycloalkylmethoxy, or 1-2C-alkoxy which is completely or predominantly substituted by fluorine,
R3 is - CₙH₂ₙ-C(O)R4, in which
R4 is phenyl, naphthyl, or phenyl substituted by R41, R42 and R43, where
R41 is halogen, 1-4C-alkyl, 1-4C-alkoxy or amino,
R42 is hydrogen, halogen or 1-4C-alkoxy,
R43 is hydrogen or halogen,
n is an integer from 1 to 2,
and the salts of these compounds.

4. Compounds of the formula I according to claim 1, in which
R1 is methoxy or ethoxy,
R2 is methoxy, ethoxy, difluoromethoxy or cyclopentyloxy,
R3 is -CₙH₂ₙ-C(O)R4, in which
R4 is phenyl, naphthyl, or phenyl substituted by R41, R42 and R43, where
R41 is amino, chlorine, methyl or methoxy,
R42 is hydrogen, chlorine or methoxy,
R43 is hydrogen or chlorine,
n is 1,
and the salts of these compounds.

5. Medicaments containing one or more compounds according to claim 1 together with the usual pharmaceutical auxiliaries and/or carrier materials.

6. Compounds according to claim 1 for use in the treatment of illnesses.

7. Use of compounds according to claim 1 for the production of medicaments for the treatment of airway disorders.

8. Use of compounds according to claim 1 for the production of medicaments for the treatment of dermatoses.
